# EUROPEAN PATENT APPLICATION

(11) **EP 2 135 609 A1**
(43) Date of publication of application: **23.12.2009**
(21) Application number: 09167139.6
(22) Date of filing: 03.11.2005
(51) Int. Cl.: A61K 31/46, A61K 45/06

(54) **Combinations of nicotinic acetylcholine alpha 7 receptor agonists**

(30) Priority: 05.11.2004 GB 0424564
(62) Divisional of application: 05799805.6
(71) Applicant: Novartis AG, 4056 Basel (CH)
(72) Inventor: Kalkman, Hans O., 4002, Basel (CH); Nozulak, Joachim, 4002, Basel (CH); Vassout, Annick, 68510, Sierentz (FR); Hurth, Konstanze, 4002, Basel (CH); Feuerbach, Dominik, 4002, Basel (CH); Gentsch, Conrad, 4102, Binningen (CH); Bilbe, Graeme, 4002, Basel (CH)
(74) Representative: Wiessner, Michael

(57) **Abstract**

combinations which comprise at least one nicotinic acetylcholine alpha 7 receptor agonist and at least one compound selected from the group consisting of (a) conventional antipsychotics (b) atypical antipsychotics (c) cognition, attention and/or memory enhancers (d) and to use of these combinations in the treatment of psychiatric disorders.

## Description

The present invention relates to combinations suitable for the treatment of psychiatric disorders.

Surprisingly, it has been found that the effect of a combination which comprises at least one nicotinic acetylcholine alpha 7 receptor agonist and at least one compound selected from the group consisting of (a) conventional antipsychotics (b) atypical antipsychotics (c) cognition, attention and/or memory enhancers (d) antidepressiva is greater than the additive effect of the combined drugs in the treatment of psychiatric disorders.

Hence, the invention relates to a combination, such as a combined preparation or pharmaceutical composition, which comprises at least one nicotinic acetylcholine alpha 7 receptor agonist and at least one compound selected from the group consisting of (a) conventional antipsychotics , (b) atypical antipsychotics and (c) cognition, attention and/or memory enhancers, in which the active ingredients are present in each case in free form or in the form of a pharmaceutically acceptable salt and optionally at least one pharmaceutically acceptable carrier; for simultaneous, separate or sequential use.

The term "psychiatric disorders" as used herein includes, but is not limited to schizophrenia, anxiety disorders, depression and bipolar disorders. Preferably, the psychiatric disorder to be treated with the combination disclosed herein is schizophrenia, more preferably schizophrenia which is refractory to monotherapy employing one of the combination partners alone.

The term "nicotinic acetylcholine alpha 7 receptor agonists" as used herein includes, but is not limited to the compounds disclosed in W0011085727, WO2004/022556, WO01/060821, WO01/29034, WO2004/016608 and. The content of these documents is incorporated herein by reference.

Preferred nicotinic acetylcholine alpha 7 receptor agonists are selected from the group consisting of compounds of formula (Ia) (as described in WO01/085727) 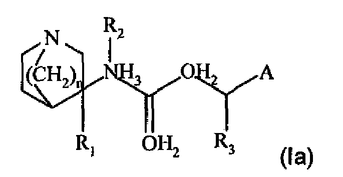 wherein n is 1 or 2,
R₁, R₂ and R₃, independently, are hydrogen or (C₁₋₄)alkyl and A is a group of formula 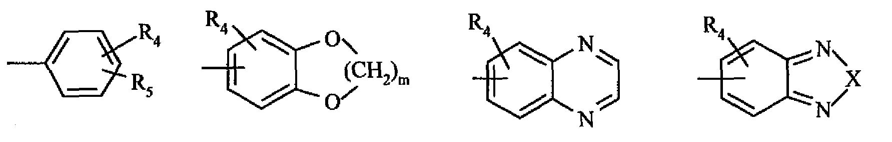 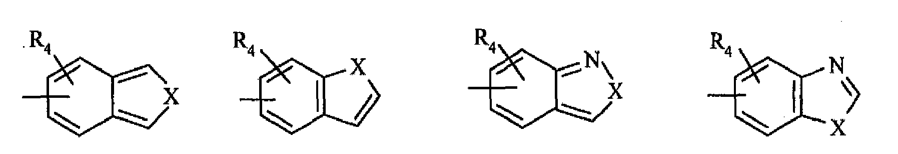 wherein m is 1, 2 or 3, X is O, S, NH or CH₂ and R₄ and R₅, independently, are hydrogen, halogen, hydroxy, (C₁₋₄)alkyl, (C₁₋₄)alkoxy, (C₁₋₄)alkylthio,
(C₁₄)alkylamino, nitro, trifluoromethyl or phenyl,
in free base or acid addition salt form.

Further, preferred nicotinic acetylcholine alpha 7 receptor agonists are selected from the group consisting of compounds of formula (Ib) (as described in WO04/022556) 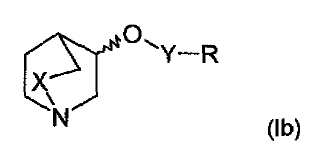 wherein
X is CH₂ or a single bond;
Y is a group of formula 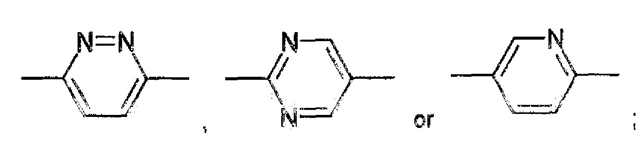 R is a substituted or unsubstituted C₅-C₁₀aryl or substituted or unsubstituted hetero-C₅-C₁₀aryl, N(R¹)(R⁴), or N(R²)(CHR³R⁴)_{;}
each of R¹, R² and R³ is independently H, C₁-C₄alkyl, or CF₃;
R⁴ is a substituted or unsubstituted C₅-C₁₀aryl or substituted or unsubstituted hetero-C₅-C₁₀aryl;
in free base or acid addition salt form.

Further, preferred nicotinic acetylcholine alpha 7 receptor agonists are selected from the group consisting of compounds of formula (Ic) (as described inWO01/60821) 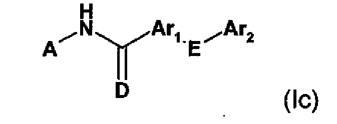 wherein:
- A: represents:

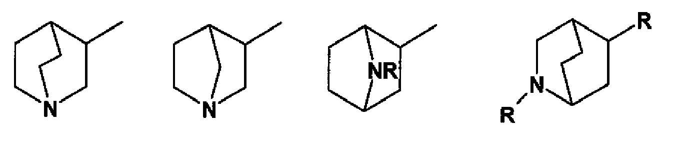

- D: represents oxygen or sulfur;
- E: represents a single bond, oxygen, sulfur, or NR10;
- R: represents hydrogen or methyl;
- Ar1: represents a 5- or 6-membered aromatic or heteroaromatic ring containing zero to three nitrogen atoms, zero or one oxygen atom, and zero or one sulfur atom:
- Ar2: represents a 5- or 6-membered aromatic or heteroaromatic ring containing zero to three nitrogen atoms, zero or one oxygen atom, and zero or one sulfur atom, or; an 8-, 9- or 10-membered fused aromatic or heteroaromatic ring system containing zero to three nitrocren atoms, zero to one oxygen atom, and zero to one sulfur atom;
wherein when Ar2 is unsubstituted phenyl, ArI is not pyrazolyl;
the aromatic rings ArI and Ar2 optionally substituted with one to three substituents selected from: halogen, C1-4alkyl, C2-4alkenyl, C2-4alkynyl, CN, NO2, NR1R2,
CH2NR1'R2, OR3. CH2,OR3, CO2R4, and CF3; but if Ar1 is phenyl and Ar2 is quinolynyl, then Ar2 is substituted with 0, 1, 2 or 3 substituents selected from C1-4alkyl,
C2- 4alkenyl, C-1-4alkynyl, CN, NO2, NR1R2, CH2NR1'R2, OR3, CH20R3 and C02R4;
R1, R2, and R3 are independently C1-4alkyl, aryl, heteroaryl, C(O)R5, C(O)NHR6,
C(O)R7, S02R8 or R1 and R2 may together be (CH2)jG(CH2)k where G is oxygen, sulfur, NR9, or a bond;
j is 2 to 4;
k is 0 to 2;
R4. R5, R6, R7, R8, R9, and R 10, are independently C 1 -4alkyl, aryl, or heteroaryl.
or an enantiomer thereof, and pharmaceutically acceptable salts thereof, with the provisos that:
if D represents oxygen, E represents a single bond, and A represents: 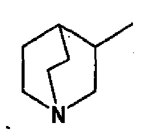 and either Ar1 or Ar2 represents a pyrazole ring, then all optional substituents on the pyrazole ring are hydrogen; and if ArI represents a pyridine ring, Ar 2 represents an aryl ring, and A represents: 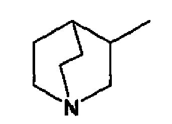 then all optional substituents on the pyridine ring shall be hydrogen, and formula I does not represent:

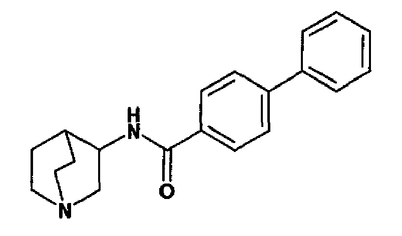

Further, preferred nicotinic acetylcholine alpha 7 receptor agonists are selected from the group consisting of compounds of formula (Id) (as described in WO01/29034) 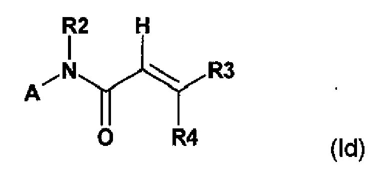 wherein
A represents: 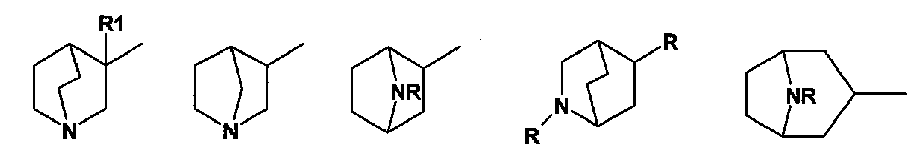 R represents hydrogen or methyl;
R1 and R2 are independently hydrogen, or C1-C4 alkyl;
R3 and R4 are independently hydrogen, C1-C4 alkyl or SAr, provided that at least one of R3 and R4 represents SAr;
Ar represents a 5- or 6-membered aromatic or heteroaromatic ring containing zero to three nitrogen atoms, zero or one oxygen atom, and zero or one sulfur atom or an 8-, 9- or 10membered fused aromatic or heteroaromatic ring system containing zero to four nitrogen atoms. zero to one oxygen atom, and zero to one sulfur atom which may optionally be substituted with one or more substituents selected from: hydrogen, halogen, C1-C4 alkyl, C-)- C4 alkenyl, C2-C4 alkynyl, aryl, heteroaryl, -C02R5, -CN, _N02, _NR6 R7, - CF3, -OR8-R5, R6, R7, and R8 are independently hydrogen, C1-C4 alkyl, aryl, heteroaryl, -C(O)R9,-C(O)NHR'O, -C(O)R", -S02R 12, or,
R6 and R7 may together be (CH2)ⱼ Q(CH2)k where Q is 0, S, NR13, or, a bond;
j is 2 to 7;
k is 0 to 2;
R9, R10, R11, R 12, and R13 are independently C1-C4 alkyl, aryl, or heteroaryl;
or an enantiomer thereof, and the pharmaceutically acceptable salts thereof.

Further, preferred nicotinic acetylcholine alpha 7 receptor agonists are selected from the group consisting of compounds of formula (Ie) (as described in WO04/016608) 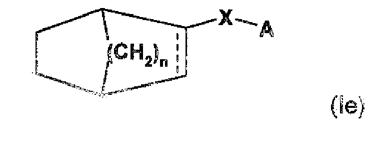 an enantiomer thereof, or a mixture of its enantiomers, or a pharmaceutically-acceptable addition salt thereof, or an onium salt thereof, wherein
------ represents an optional double bond;
n is 1, 2 or3;
X represents a linker selected from -0-, -O-CH2-, -O-CH2-CH2-, -S-, -SO-, -SO2-, -CH2-, -S-CH2- CH2-, -CH2-, -C(=CH2)-,-NH-, -N(alkyl)-, -C(=O)-, -C(=S)-, 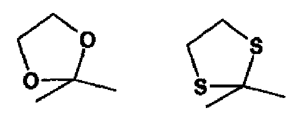 and
A represents a monocyclic or polycyclic, carbocyclic or heterocyclic group, optionally substituted one or more times with substituents selected from the group consisting of alkyl, cycloalkyl, cycloalkyl-alkyl, alkoxy, hydroxyalkoxy, alkoxy-alkyl, alkoxy-alkoxy, cycloalkoxy, cycloalkoxy-alkyl, cycloalkoxy-alkoxy, halo, CF3, CN, NO2, 25 NH2, carboxy, carbamoyl, amido, sulfamoyl, and phenyl, or with another monocyclic or polycyclic, carbocyclic or heterocyclic group, which additional monocyclic or polycyclic, carbocyclic or heterocyclic group may optionally be substituted one or more times with substituents selected from the group consisting of alkyl, cycloalkyl, cycloalkyl-alkyl, provided however if X represents O or S, than A is a biphenyl group.

Particularly preferred nicotinic acetylcholine alpha 7 receptor agonists are selected from the group consisting of the following compounds
I.1 (S)-(1-Aza-bicyclo[2.2.2]oct-3-yl)-carbamic acid (S)-1-(2-fluoro-phenyl)-ethyl ester
I.2 (R)-(1Aza-bicyclo[2.2.2]oct-3-yl)-carbamic acid (R)-1-(2-chloro-phenyl)-ethyl ester
I.3 (S)-(1-Aza-bicyclo[2.2.2]oct-3-yl)-carbamic acid (S)-1-phenyl-ethyl ester
I.4 (R)-3-[6-(2-Fluoro-4-methyl-phenyl)-pyridazin-3-yloxy]-1-aza-bicyclo[2.2.2]octane
I.5 (R)-3-[6-(2,5-Difluoro-4-methyl-phenyl)-pyridazin-3-yloxy]-1-azabicyclo[2.2.2]octane
I.6 (R)-3-(6-p-Tolyl-pyridin-3-yloxy)-1-aza-bicyclo[2.2.2]octane
I.7 (R)-3-[6-(3,4-Dimethyl-phenyl)-pyridazin-3-yloxy]-1-aza-bicyclo[2.2.2]octane
I.8 (R)-3-(6-p-Tolyl-pyridazin-3-yloxy)-1-aza-bicyclo[2.2.2]octane
I.9 (R)-3-[5-(2-Fluoro-4-methyl-phenyl)-pyrimidin-2-yloxy]-1-aza-bicyclo[2.2.2]octane
I.10 (2S,3R)-3-[6-(1H-Indol-5-yl)-pyridazin-3-yloxy]-2-methyl-1-azabicyclo[2.2.2]octane
I.11 (2S,3R)-2-Methyl-3-[6-(5-methyl-thiophen-2-yl)-pyridazin-3-yloxy]-1-azabicyclo[2.2.2]octane
I.12 3-[6-(2,3-Dimethyl-1H-indol-5-yl)-pyridazin-3-yloxy]-2-methyl-1-azabicyclo[2.2.2]octane
I.13 3-[6-(1H-Indol-5-yl)-pyridin-3-yloxy]-2-methyl-1-aza-bicyclo[2.2.2]octane
I.14 4-[5-(1H-Indol-5-yl)-pyrimidin-2-yloxy]-1-aza-bicyclo[3.3.1]nonane
I.15 4-[5-(1H-Indol-5-yl)-pyridin-2-yloxy]-1-aza-bicyclo[3.3.1]nonane
I.16 5-Benzofuran-5-ylethynyl-1-methyl-3-piperidin-1-ylmethyl-pyrrolidin-2-one
I.17 1-Methyl-5-phenylethynyl-3-piperidin-1-ylmethyl-pyrrolidin-2-one
I.18 1-Methyl-5-(1-methyl-1H-indol-5-ylethynyl)-3-piperidin-1-ylmethyl-pyrrolidin-2-one
I.19 5-(3-Amino-phenylethynyl)-1-methyl-3-piperidin-1-ylmethy-pyrrolidin-2-one

Very particularly preferred nicotinic-alpha 7 receptor agonists are selected from the group consisting of the following compounds:

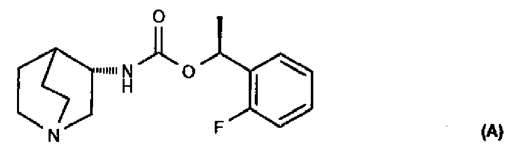

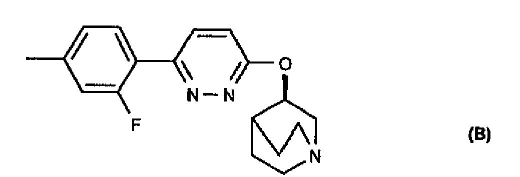

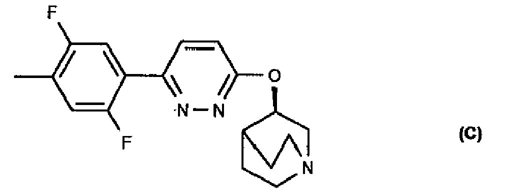

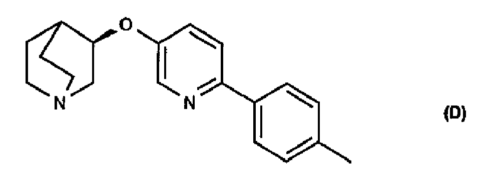

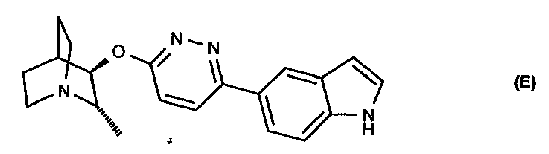

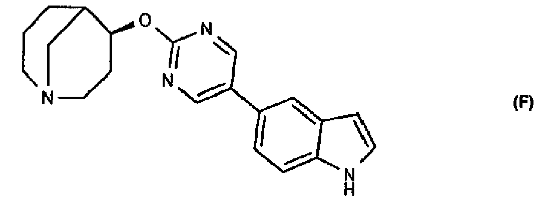

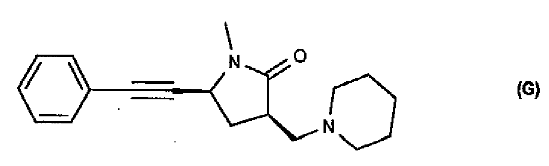

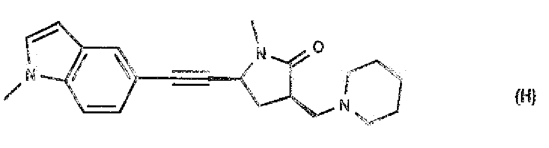

The term "conventional antipsychotics" denotes compounds that are effective in treating psychoses mainly via dopamine receptor D2 antagonism. "Conventional antipsychotics" as used herein includes, but is not limited to haloperidol, droperidol, molindone, fluphenazine, thiotixene, flupentixol, promazine, pimozide, chlorpromazine, methotrimeprazine, pipotiazine trifluoperazine, thioridazine, acetophenazine, chlorprothixene and mesoridazine.

The term "atypical antipsychotics" denotes compounds that are effictive in treating psychoses via an additional and/or different mechanism than dopamine receptor 2 antagonsim. "Atypical antipsychotics" as used herein includes, but is not limited to clozaril, risperidone, olanzapine, quetiapine, ziprasidone, aripiprazol, sertindole, perphenazine, mesoridazine, prochlorperazine, naproxene and loxapine.

The term "cognition enhancer" denotes compounds that improve any type of memory and attention (short term, long term, working memory, implicit and/or explicit memory, episodic primary and secondary memory) Cognition enhancer, as used herein includes, but is not limited to cholinergic compounds like acetylcholineesterase inhibitors and/or buturylesterase inhibitors (rivastigmine, donezepil, galantamine, huperzine), ampakines (e.g. CX614, CX516), muscarinic modulators (e.g. muscarinic receptor agonists), modulators of the NMDA-receptor (e.g. positive modulators, antagonists, memantine), agonists and/or positive modulators of nicotinic acetylcholine receptors, phosphodiesterase inhibitors (e.g. PDE4 inhibitors), nootropic compounds like hydergine, oxiracetam, aniracetam, acetyl-L-carnitine, ginko-derived compounds, compounds contained in gerovitals like p-aminobenzoic acid and dithylaminoethanol and derivative thereof and attention-modulating compounds like methylphenicate, tomoxetine and modafinil.

The term "antidepressiva" denotes a drug that has been shown to improve depressive symptoms. "Antidepressiva" as used herein includes, but is not limited to the following groups: 1) heterocyclics like imipramine, desipramine, amitriptyline, nortriptyline, doxepine, maprotiline; 2) monoamine-oxidase inhibitors, including reversible MAO-inhibitors like phenelzine, isocarboxazid, tranylcypromine, moclobemide; 3) monoamine-uptake inhibitors (specific, dual or triple) like fluoxetine sertraline, reboxetine, duloxetine, paroxetine and bupropione; 4) HPA-axis modulating compounds like CRF modulators (e.g. CRF antagonists), glucocorticoid receptor modulators (e.g. GR antagonists), mineralcorticoid modulators (e.g. MR antagonists); 5) neuropeptide modulators like galanin receptor modulators (e.g. GalR 3 antagonists, GalR1 agonists, GalR2 agonists), vasopressin receptor modulators (e.g. V1a, V1b antagonists), somatostatin receptor modulators (e.g. SSTR1 and/or SSTR3 antagonists), tachykinergic modulators like NK1 and/or NK2 receptors modulators (e.g. NK1 antagonists), NPY receptor modulators; 6) voltage gated calcium channel modulators (e.g. gabapentine); 7) sigma site modulators; 8) melatoninergic compounds (e.g. melatonin, agomelatine); 9) modulators of the metabotropic glutamate receptors (e.g. mGluR5 antagonists, mGluR7 antagonists); 10) modulators of the mitochondrial benzodiazepine receptor (e.g. agonists of the MDR); 11) modulators of the nicotinic acetylcholine receoptors (e.g. alpha4beta2 and/or alpha3beta4 subtype containing antagonists); 12) Lithium ; 13) antidepressant treatments like sleep deprivation, electroshock; 14) natural products, such as extracts of *hypericum perforatum* and derivatives.

The term "anxiolytics" denotes compounds that block or alleviate anxiety and/or emotional tension and restore the clinically anxious individual to better tolerate its level of anxiety and/or prevents the progression into an anxiety-episode. The vaste majority of the compounds and pharmacological principles mentioned under "Antidepressiva" are also indicated under "Anxiolytics". "Anxiolytics", as used herein additionally includes, but is not limited to benzodiazepines, azapirones, meprobamate, barbiturates and certain anthistamines such as diphenydramine and hydroxyzine, furthermore chlordiazepoxide, demoxepam, chlorazepate, medazepam, diazepam, temazepam, oxazepam, prazepam, alprazolam, buspirone, zolpidem, flurazepam, lorazepam, triazolam, quazepam, clonazepam.

In a preferred embodiment, the combination according to the invention comprises one nicotinic acetylcholine alpha 7 receptor agonist and one conventional antipsychotic.

In a preferred embodiment, the combination according to the invention comprises one nicotinic acetylcholine alpha 7 receptor agonist and one atypical antipsychotic.

In a preferred embodiment, the combination according to the invention comprises one nicotinic acetylcholine alpha 7 receptor agonist and one cognition, attention and/or memory enhancer.

In a preferred embodiment, the combination according to the invention comprises one nicotinic acetylcholine alpha 7 receptor agonist and one antidepressivum.

In a particularly preferred embodiment, the combination according to the invention comprises compound (A) and one or more, preferably one, compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene, diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

In a particularly preferred embodiment, the combination according to the invention comprises compound (B) and one or more, preferably one compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene, diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

In a particularly preferred embodiment, the combination according to the invention comprises compound (C) and one or more, preferably one compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene, diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

In a particularly preferred embodiment, the combination according to the invention comprises compound (D) and one or more, preferably one compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene , diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

in a particularly preferred embodiment, the combination according to the invention comprises compound (E) and one or more, preferably one compound selected .from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene, diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

In a particularly preferred embodiment, the combination according to the invention comprises compound (F) and one or more, preferably one compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene, diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

In a particularly preferred embodiment, the combination according to the invention comprises compound (G) and one or more, preferably one compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene, diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

In a particularly preferred embodiment, the combination according to the invention comprises compound (H) and one or more, preferably one compound selected from the group consisting of agomelatine, azapirones, alprazolam, amitriptyline, aniracetam, acetyl-L-carnitine, aripiprazol, acetophenazine, benzodiazepines, barbiturates, buspirone, bupropione;chlordiazepoxide, chlorazepate, clonazepam, chlorpromazine, clozaril, CX614, CX516, chlorprothixene , diphenydramine hydroxyzine, demoxepam, diazepam, droperidol, duloxetine, donezepil, doxepine, desipramine, flurazepam, fluphenazine, fluoxetine, flupentixol, gabapentine, melatonin, ginko-derived compounds, galantamine, haloperidol, hydergine, huperzine, isocarboxazid, imipramine, lorazepam, loxapine, meprobamate, medazepam, moclobemide; molindone, maprotiline; modafinil, memantine, methylphenicate, mesoridazine, mesoridazine, methotrimeprazine, nortriptyline, naproxene, oxazepam, oxiracetam, olanzapine, prazepam, paroxetine, phenelzine, pipotiazine, perphenazine, promazine, pimozide, PDE4 inhibitors, quazepam, quetiapine, reboxetine, rivastigmine, rochlorperazine, risperidone, sertraline, sertindole, temazepam, triazolam, tranylcypromine, tomoxetine, thiotixene, trifluoperazine, thioridazine, zolpidem, ziprasidone.

The term "psychiatric disorders" is known to the expert and includes all disorders the expert in the field would consider. In particular, schizophrenia, anxiety disorders and bipolar disorders are included.

In a further embodiment, the combinations of the invention can be used to treat schizophrenia which is refractory to monotherapy employing one of the combination partners alone.

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo*.

Haloperidol can be administered, e.g., in the form as marketed, e.g. under the trademark Haloperidol STADA™. Fluphenazine can be administered, e.g., in the form of its dihydrochloride as marketed, e.g. under the trademark Prolixin™. Thiothixene can be administered, e.g., in the form as marketed, e.g. under the trademark Navane™. It can be prepared, e.g., as described in US 3,310,553. Flupentixol can be administered for instance in the form of its dihydrochloride, e.g., in the form as marketed, e.g. under the trademark Emergil™ or in the form of its decanoate, e.g., in the form as marketed, e.g. under the trademark Depixol™. It can be prepared, e.g., as described in BP 925,538. Clozaril can be administered, e.g., in the form as marketed, e.g. under the trademark Leponex™. It can be prepared, e.g., as described in US 3,539,573. Risperidone can be administered, e.g., in the form as marketed, e.g. under the trademark Risperdal™. Olanzapine can be administered, e.g., in the form as marketed, e.g. under the trademark Zyprexa™. Quetiapine can be administered, e.g., in the form as marketed, e.g. under the trademark Seroquel™. Ziprasidone can be administered, e.g., in the form as marketed, e.g. under the trademark Geodon™. It can be prepared, e.g., as described in GB 281,309. Aripiprazole can be administered, e.g., in the form as marketed, e.g. under the trademark Abilify™. It can be prepared, e.g., as described in US 5,006,528.

The term "a combined preparation", as used herein defines especially a "kit of parts" in the sense that the first and second active ingredient as defined above can be dosed independently or by use of different fixed combinations with distinguished amounts of the ingredients, i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the active ingredients. The ratio of the total amounts of the active ingredient 1 to the active ingredient 2 to be administered in the combined preparation can be varied, e.g., in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the first and second active ingredient, in particular a synergism, e.g. a more than additive effect, additional advantageous effects, less side effects, a combined therapeutically effect in a non-effective dosage of one or both of the first and second active ingredient, and especially a strong synergism the first and second active ingredient.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in form of a hydrate or include other solvents used for crystallization.

A combination which comprises at least one nicotinic acetylcholine alpha 7 receptor agonist ("compound i)") and at least one compound selected from the group consisting of (a) conventional antipsychotics and (b) atypical antipsychotics ("compound ii)"), in which the active ingredients are present in each case in free form or in the form of a pharmaceutically acceptable salt, if at least one salt-forming group is present, will be referred to hereinafter as a COMBINATION OF THE INVENTION.

Surprisingly it was found that the administration of a COMBINATION OF THE INVENTION results in a beneficial, especially a synergistic, therapeutic effect or in other surprising beneficial effects, e.g. less side effects, compared to a monotherapy applying only one of the pharmaceutically active ingredients used in the COMBINATION OF THE INVENTION.

The therapeutic potential of a COMBINATION OF THE INVENTION may, for example, be evidenced in preclinical studies known as such, e.g. the methods mentioned or described herein.

The antipsychotic potential of the COMBINATION OF THE INVENTION can be indicated in standard tests, e.g. in the ketamine-induced hyperlocomotion test or the object recognition test or in clinical studies.

### ketamine-induced hyperlocomotion

Blockade of ketamine-induced hyperlocomotion is well known as screening paradigm for antischizophrenic activity, especially for positive symptoms observed with schizophrenia. Male rats are used. In principle 8 treatment groups are formed:
1) nicotinic acetylcholine alpha 7 receptor agonists followed by solvent 2 and solvent 3 to study the effects of the nicotinic acetylcholine alpha 7 receptor agonists on locomotor activity.
2) Solvent 1, combination partner and solvent 3 to study the effects of the combination partner on locomotor activity.
3) Solvent 1, solvent 2, followed by ketamine (1 mg/kg) to study the induction of hyperlocomotor activity.
4) nicotinic acetylcholine alpha 7 receptor agonists followed by solvent 2 and ketamine.
5) Solvent 1 followed by combination partner and ketamine (1 mg/kg).
6) The COMBINATION OF THE INVENTION (doses of each active ingredients at doses close to threshold) followed by solvent 3.
7) The COMBINATION OF THE INVENTION (doses of each active ingredients at doses close to threshold) followed by ketamine (1 mg/kg).
8) Solvent 1 - solvent 2 - solvent 3.
   Rats are randomly allocated to these pretreatment groups (n=10 / dose group). Drugs are administered subcutaneous (s.c.), 15 min prior to ketamine. Immediately after the animals received ketamine, they are placed into the activity monitor for a period of 60 min. Locomotor activity is analysed over the initial 30 minutes.

Locomotion is recorded with a videotracking system. Animals are on a normal 12/12 h. day-night cycle, with light on at 06:00 H. Experiments are performed in a dimly lit room between 07:00 H and 15:00 H. Animals are placed in a round arena (diameter 42 cm, height 32 cm) made of grey polyvinylchloride plastic. The camera is placed such, that four animals (one per arena) can be recorded simultaneously.
Ketamine is dissolved in physiological saline as 1 mg/ml and administered s.c. in a volume of 1 ml/kg. The COMBINATION OF THE INVENTION is dissolved as required. It is administered s.c. in a volume of 1 ml/kg.
Comparison between groups is done with Student's t-test, corrected for multiple testing using the Bonferroni procedure.

### object recognition test

Another suitable preclinical test demonstrating the antipsychotic potential of the COMBINATION OF THE INVENTION is an object recognition test, e.g. the test described below. In the test described below the potential of COMBINATION OF THE INVENTION for its effects on attention and/or memory in mice using the object recognition test (ORT), in which neither positive (e.g. food reward) nor negative (e.g. shock) reinforcement is applied, can be compared to the potential of the combination partners.

Method. When inspecting novel objects, rodents repeatedly adopt a characteristic posture, the Stretched Attend Posture (SAP) which they direct towards the object. The Object Recognition Test records the number of SAP shown by singly-housed mice towards a PVC disc placed in their home cage for 3 minutes on 3 successive occasions (0 minute, 10 minutes and 24 h), To test for any potential motor disturbances or decline in motivation after drug treatment, all mice are presented, in addition, with a novel PVC cone at 24 h 10 minutes.

### clinical study

Furthermore, the pharmacological activity of a COMBINATION OF THE INVENTION may, for example, be demonstrated in a clinical study. In one embodiment of the present invention, such clinical studies are preferably randomized, double-blind, clinical studies in patients with schizophrenia. Such studies demonstrate, in particular, the synergism of the active ingredients of the COMBINATIONS OF THE INVENTION. The beneficial effects on schizophrenia can be determined directly through the results of these studies or by changes in the study design which are known as such to a person skilled in the art. The studies are, in particular, suitable to compare the effects of a monotherapy using the active ingredients and a

### COMBINATION OF THE INVENTION.

The COMBINATIONS OF THE INVENTION provide in one embodiment, in particular, benefits in the treatment of positive symptoms, negative symptoms, mood symptoms and/or cognitive symptoms of schizophrenia and/or psychosis. Furthermore, some of the COMBINATIONS OF THE INVENTION show beneficial effects in the control of impulsive and/or violent behavior of schizophrenic patients.

A further benefit is that lower doses of the active ingredients of the COMBINATION OF THE INVENTION can be used, for example, that the dosages need not only often be smaller but are also applied less frequently, or can be used in order to diminish the incidence of side effects. This is in accordance with the desires and requirements of the patients to be treated.

It is one objective of this invention to provide a pharmaceutical composition comprising a quantity, which is jointly therapeutically effective against psychiatric disorders, of a COMBINATION OF THE INVENTION and at least one pharmaceutically acceptable carrier. In this composition, the first and second active ingredient can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to humans, comprising a therapeutically effective amount of at least one pharmacologically active ingredient, alone or in combination with one or more pharmaceutically acceptable carries, especially suitable for enteral or parenteral application. The preferred route of administration of the dosage forms of the present invention is orally.

The novel pharmaceutical composition contains, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of active ingredient or ingredients contained in an individual dose of each dosage form need not in itself constitute an effective amount since the necessary effective amount can be reached by administration of a plurality of dosage units.

In preparing the compositions for oral dosage form, any of the usual pharmaceutical media may be employed, such as, for example, water, glycols, oils or alcohols; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form in which case solid pharmaceutical carriers are obviously employed.

Furthermore, the present invention relates to the use of a COMBINATION OF THE INVENTION comprising at least one conventional antipsychotic or at least one atypical antipsychotic in combination with at least one nicotinic acetylcholine alpha 7 receptor agonist for the preparation of a medicament for the treatment of schizophrenia.

Additionally, the present invention provides a method of treating a human patient having at least one psychiatric disorder comprising administering to the patient a COMBINATION OF THE INVENTION in a quantity which is jointly therapeutically effective against psychiatric disorders and in which the compounds can also be present in the form of their pharmaceutically acceptable salts.

Moreover, the present invention provides a commercial package comprising as active ingredients COMBINATION OF THE INVENTION, together with instructions for simultaneous, separate or sequential use thereof in the treatment of psychiatric disorders.

In particular, a therapeutically effective amount of each of the active ingredients of the COMBINATION OF THE INVENTION may be administered simultaneously or sequentially and in any order, and the components may be administered separately or as a fixed combination. For example, the method of treatment of schizophrenia according to the invention may comprise (i) administration of the first active ingredient in free or pharmaceutically acceptable salt form and (ii) administration of the second active ingredient in free or pharmaceutically acceptable salt form, simultaneously or sequentially in any order, in jointly therapeutically effective amounts, preferably in synergistically effective amounts, e.g. in daily dosages corresponding to the amounts described herein. The individual active ingredients of the COMBINATION OF THE INVENTION can be administered separately at different times during the course of therapy or concurrently in divided or single combination forms. Furthermore, the term administering also encompasses the use of a pro-drug of an active ingredient that convert *in vivo* to the active ingredient. The instant invention is therefore to be understood as embracing all such regimes of simultaneous or alternating treatment and the term "administering" is to be interpreted accordingly.

In one preferred embodiment of the invention, the COMBINATION OF THE INVENTION is used for the treatment of schizophrenia which is refractory to monotherapy.

The effective dosage of each of the active ingredients employed in the COMBINATION OF THE INVENTION may vary depending on the particular compound or pharmaceutical composition employed, the mode of administration, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise. In particular,

Clozaril may be administered to an adult patient in a total daily dosage of between about 300 to about 900 mg.

Haloperidol may be administered to a patient in a total daily dosage of between about 2.5 to about 30 mg.

Olanzapine can be administered to a patient in a total daily dosage of between about 2.5 to about 20 mg.

Quetiapine can be administered to a patient in a total daily dosage of between about 500 to about 600 mg.

Risperidone may be administered to a patient in a total daily dosage of between about 2 to about 6 mg.

## Claims

1. A combination which comprises i) one or more nicotinic acetylcholine alpha 7 receptor agonists and ii) one or more compound s selected from the group (a) conventional antipsychotics (b) atypical antipsychotics (c) cognition, attention and/or memory enhancers (d) antidepressiva.

2. Combination according to claim 1 which is a combined preparation or a pharmaceutical composition.

3. Combination according to any one of claims 1 to 2 for simultaneous, separate or sequential use for the treatment of psychiatric disorders.

4. Combination according to claim 3, wherein the disorder is schizophrenia.

5. Combination according to claim 3, wherein the disorders are anxiety disorders or bipolar disorders.

6. Use of a combination according to claim 1 or 2 for the preparation of a medicament for the treatment a psychiatric disorder.

7. Use of a combination according to claim 1 or 2 for the preparation of a medicament for the treatment of schizophrenia.

8. Use of a combination according to claim 1 or 2 for the preparation of a medicament for the treatment of anxiety disorders or bipolar disorders.

9. Method of prevention, treatment or delay of progression of a psychiatric disorder in a patient in need thereof comprising administering to said patient an effective amount of a combination according to claim 1.

10. Method of prevention, treatment or delay of progression of a shizophrenia in a patient in need thereof comprising administering to said patient an effective amount of a combination according to claim 1.

11. Method of prevention, treatment or delay of progression of a anxiety disorders or bipolar disorders in a patient in need thereof comprising administering to said patient an effective amount of a combination according to claim 1.

12. A commercial package comprising a combination according to claim 1 or 2 together with instructions for simultaneous, separate or sequential use thereof in the treatment of a psychiatric disorder.

13. A combination which comprises at least one nicotinic acetylcholine alpha 7 receptor agonist and at least one compound selected from the group consisting of ((a) conventional antipsychotics (b) atypical antipsychotics (c) cognition, attention and/or memory enhancers (d) antidepressiva, in which the active ingredients are present in each case in free form or in the form of a pharmaceutically acceptable salt and optionally at least one pharmaceutically acceptable carrier; for simultaneous, separate or sequential use.

14. A pharmaceutical composition comprising a quantity, which is jointly therapeutically effective against at least one psychiatric disorder of a pharmaceutical combination according to claim 1 or 2 and at least one pharmaceutically acceptable carrier or diluent.
